# EUROPEAN PATENT APPLICATION

(11) **EP 1 335 270 A1**
(43) Date of publication of application: **13.08.2003**
(21) Application number: 03011085.2
(22) Date of filing: 29.10.1999
(51) Int. Cl.: G06F 3/00, G06F 19/00, G10L 15/26

(54) **Non-manual control of a medical image display station**

(30) Priority: 30.10.1998 US 106392 P; 24.06.1999 US 339427; 29.10.1999 US 430514
(62) Divisional of application: 99971534.5
(71) Applicant: AMD Industries LLC, Hawthoren, CA 90250 (US)
(72) Inventor: Geisberg, Daniel Jay, Los Angeles, CA 90049 (US); Lappen, David Irwin, Santa Monica, CA 90402 (US); Lappen, William Ascher, Encino, CA 91436 (US)
(74) Representative: HOFFMANN - EITLE

(57) **Abstract**

A method and system for using non-manual commands, preferably voice commands, to control the actions of a medical image digital display system, eliminate certain job tasks and facilitate a work environment with minimum changes while providing the advantages of digital manipulation. Images are provided to the medical image digital display system to be displayed and controlled by a computer system using non-manual commands that do not require the use of the limbs of the hands or feet. The image may include patient data, history, test data and information in place of or in addition to the image, which can include an X-ray, CT scan, MRI scan or other types of images.

## Description

### CROSS-REFERENCE TO RELATED APPLICATION

This application claims the benefit of U.S. Provisional Application Number 60/106,392, filed October 30, 1998, which application is specifically incorporated herein, in its entirety, by reference; the present application is also a continuation in part of copending application Serial No. 09/339,427, entitled "Display Device for Both Hard Copy and Digital Images" which was filed on June 24, 1999 and is incorporated herein by reference.

### BACKGROUND OF THE INVENTION

### 1. Field of the Invention

The present invention relates to medical image display systems, and more particularly, to a method and apparatus for using non-manual control, such as voice, to control a Medical Image Display Station.

### 2. Description of Related Art

Physicians use X-Ray, Magnetic Resonance Imaging (MRI), Computer Aided Tomography (CAT) Scans and other diagnostic imaging tools to create images of structures and tissues within the human body. Until recently, these images were normally stored on medical film, *i.e.,* transparent support materials, intended to be viewed by transillumination on a "view box". Many modern diagnostic systems create and store images without the use of film and can feed the image data directly to digital viewing stations. These stations consist of one or more monitors connected to a computer system and have the ability to control, manipulate and/or adjust the image(s) being displayed. In addition to directly handling digital images from digital systems, traditional film-based images can be scanned for display on digital viewing stations.

There are at least four reasons that radiologists and hospital information managers often favor traditional film viewing stations over digital workstations:
1. A physician can not readily view as many patient images on a workstation as may be required for proper diagnosis;
2. Image presentation and manipulation on a workstation tends to be cumbersome and switching to the next patient's films (images) takes too long;
3. The workstation presents images in a manner that is counterintuitive and opposite to traditional work flow; and
4. Digital technology appears to be too expensive.

That is, digital viewing stations have been slow to gain widespread acceptance, partially due to performance (speed) and partially because their use imposes work flow patterns not consistent with current practices. Further, the reliance on non-standard and non-intuitive icons presents barriers to new users. In the diagnostic process, the physician must quickly and accurately diagnose and interpret the medical condition from the displayed image. Speed and accuracy are required for any viewing station. Current interfaces to the digital viewing stations require the physician to move his/her eye away from the medical image under study to a tool bar or memorize mouse commands or use a computer keyboard to operate the system. The so-called "softcopy" diagnosis of a digital image is currently more time consuming than the traditional reading of a "hardcopy" medical film. Although the ability to control the image under softcopy viewing can provide enhanced diagnostic information, hardcopy viewing allows the physician to process more cases per unit of time. Anything that slows down, distracts, or draws the physician away from the medical image slows the diagnostic process and creates a hurdle to market acceptance.

Although physicians may not be comfortable or familiar with a computer mouse or keyboard or other computer input devices, most are very familiar with voice dictation and are not distracted by its use. Hands free control of a viewing station also allows physicians to manipulate other documents, such as hardcopy patient records. Both dictation and handling other records are normal, expected parts of the current diagnostic process and a digital viewing system that incorporates these practices should be readily adopted by physicians.

It is known in the art to substitute voice command for traditional computer input (*e.g*., keyboards) in some medical systems. U.S. Patent No. 5,544,654 to Murphy, et al. describes a voice control of a medical ultrasound-scanning machine. This invention discloses the use of a subset of commands based on the system's current state. This technique improves reliability of the voice recognition but is little more than substitution of oral commands for typed commands. Although the above patent contains a method for controlling a machine, it is not directly applicable to a digital display station where the command structure must be compatible with patient record handling, image manipulation and the other aspects of medical film viewing explained above.

Accordingly, a need exists to provide a technique for non-manual control of a medical image viewing station. Such control may include image enhancement, image positioning and orientation as well as control over patient records.

### SUMMARY OF THE INVENTION

In accordance with the teachings of the present invention, an extremely easy and intuitive interface to the digital viewing station is provided by allowing the viewing physician to command the digital viewing station by using non-manual control features including voice commands. Rather than adding new job tasks, this invention eliminates or streamlines some tasks while providing a work environment with a minimum of changes from manipulation of traditional "hardcopy" films while still providing the advantages of digital imaging. This invention allows the viewing physician to maintain eye focus and concentration while manipulating the viewed image for improved diagnosis. Head and/or eye motion sensing hardware and software can be used by the view station to control the position of a cursor on the screen and/or command functions. "Non-manual" indicates a system that does not use limbs, either the hands or the feet for system control. The displayed information can include patient history data, test data and information in place of or in addition to the diagnostic images which can include an X-ray, CT scan, MRI scan and other types of images.

In general, the invented method, apparatus and system include the following features:
- An apparatus and system for using non-manual control, preferably voice control and/or gaze control, to change the display of medical images, comprises a device for receiving medical images; a display for showing the received medical images; and a device for controlling the display of the medical images through, at least in part, non-manual, preferably, voice commands and/or gaze control.
- The device for receiving medical images comprises at least one of a transmission medium such as wire, cable, microwave, radio wave, light wave, sound wave, or physical media that holds the images such as floppy disk, removable hard disk, CD ROM or tape.
- The display for the received medical images comprises display monitors, projection systems, or printers connected to a computer system.
- The device for controlling the display of medical images comprises at least one microphone connected to a computer capable of recognizing voice commands and a gaze detection system.
- The computer system may also be connected to a dictation system capable of recording the reading physician's diagnosis. In such a case the computer system may also be capable of recognizing dictation and converting it to machine and/or human readable text.
- For voice control purposes the computer system should be able to recognize spoken commands without prior training by that speaker. However, improved reliability results if the computer system is trained by the particular speaker to recognize that speaker's voice commands.
- The voice command feature may be deactivated at will and the system controlled by conventional means namely, manual devices including at least one of a keyboard, trackball, mouse, foot pedal, light pen, touch screen or other manual input devices. Alternatively, any of the listed manual control devices can be used along with voice command.
- The information displayed may include reports or other non-image information as well as audio playback or text of dictated remarks.
- The apparatus for using head or eye control to point to a location on a viewing station, comprises a device for receiving medical images; a display for received medical images; a gaze control device for indicating the current location of interest; and a device for controlling the movement of the current location of interest with changes in gaze, *i.e.,* with changes in head or eye position.
- The computer system is also capable of notifying the user of improper commands and instructing or suggesting other commands either by visual and/or audio cues.
- The users' impressions (oral and/or typed) or verified reports may be automatically e-mailed, together with a copy of the images analyzed, to referring physicians or wherever the user wishes.

It is an object of the present invention to provide a simple and intuitive non-manual method of controlling a medical viewing station.

Accordingly, the present invention can be incorporated into a medical viewing station to provide voice control as well as other non-manual system control.

A more complete understanding of the invention may be obtained from the detailed description that follows taken with the accompanying drawings.

### BRIEF DESCRIPTION OF THE DRAWINGS

Features, aspects and advantages of the invention will be more fully understood when considered with respect to the following detailed description, appended claims and accompanying drawings where like reference signs indicate like structures.
FIG. 1 is a schematic of a viewing station according to one embodiment of the present invention.
FIG. 2 is a typical screen interface according to an embodiment of the present invention.
FIG. 3 is a typical flow diagram of the system according to an embodiment of the present invention.
FIG. 4 is a drawing illustrating a workflow on a preferred system according to an embodiment of the present invention.

### DETAILED DESCRIPTION OF THE PREFERRED EMBODIMENT

The following preferred embodiment is an example of the application of the present invention, but is not intended to limit the invention in any way. The present invention provides a system for using non-manual commands to control the actions of a medical image display system. In the detailed description that follows, it should be appreciated that like element numerals are used to describe like elements illustrated in one or more of the figures.

The workflow model envisioned in the software allows the workstations of the present invention to greatly increase productivity. The workstation has been designed to fit into the existing workflow of a radiologist's practice. Many repetitive functions currently being performed have been automated and simplified to allow for time savings. The layout and controls use the paradigm of hanging film. As such, a technician may preview and arrange studies (ordering the film hanging, prioritizing the patients). However, a technician is not required. The software can group studies by patient, date, requested reading physician and other criteria for automatic display. In either case, the reading physician has full control over the images and their locations.

In a standard hospital-based practice, the radiologist is presented with studies of various patients, along with clinical observations for each patient. The clinical observations present the basis for the tests that have been performed and indicate the symptoms or suspected problems. If prior studies of the same patient are available, they should ideally be viewed adjacent to the current studies. With this information, the radiologist "reads" the images and dictates findings. In about 80% of the cases, the radiologist finds nothing worth commenting upon and merely indicates that he/she viewed the studies and that everything is normal. In the remainder of the cases, there will be substantive dictation required. The dictated report is typically routed to the hospital's dictation pool and then through the hospital to the referring physician or to other specialists. The radiologist may be paid on a "per read" basis or some other basis.

Digital medical images can be obtained from many sources. The actual modality generating the image might be digital (MRI, CAT) and the modality can directly feed its images to the inventive workstation. PACS (picture archiving and communications system) or other storage devices might hold digital images for transmission to the workstation. Non-digital images (film) can be digitized with a scanner. Similarly, non-standard digital images may be either printed to film for scanning into a standard digital format or may be converted to feed the inventive workstation or PACS. Once digital, the images may be transmitted to the workstation via removable media (CD-ROM, diskette or tape) or via a network.

Studies on the workstation are arranged in a "worklist". This list consists of both pending and already read studies, allowing fast retrieval of recently read studies. The pending worklist may be managed by a technician prior to the reading physician beginning a reading session. This management may include prioritizing the worklist in a manner other than the default modified first in, first out manner. A technician may also arrange the images in an order that the reading physician prefers. However, order preferences may also be entered into the workstation and will be automatically filled, where appropriate information is available with the images. The reading physician may view his/her entire worklist at any time so as to move to any patient, but the physician will probably just advance to the next patient after completing the present patient. When viewing the worklist, the physician only sees his/her worklist plus any unassigned studies. He/she may disable the unassigned study display to concentrate on his/her cases. This allows other physicians to process the unassigned cases, while only the assigned physician may read the assigned studies. The worklist continuously updates as new cases arrive. After all pending patients are analyzed, the worklist automatically appears. Once the studies have been read, the inventive workstation can easily and quickly capture the normal diagnosis (no acute abnormalities found). This represents approximately 80% of the standard hospital practice and can be accomplished with a single keystroke or command, completely eliminating dictation for most patient reports. The information that can be captured (and printed or saved) includes the date of the reading, the physician who performed the reading, the studies examined, the patient name, the referring physician, the hospital where the studies were performed and other information that normally would take up to 60 seconds to dictate per patient. It can also include any additional information provided by the original data file, HIS, RIS or DICOM data file; for example, any information provided by patient data fields in DICOM (Digital Image Communication in Medicine, a file and communications standard developed by an international committee including physicians, hardware manufacturers and software developers). This feature will save radiologists, typists and referring physicians a significant amount of time. The workstation may route this normal diagnosis, as well as other diagnoses, as required back to the referring department, to a hospital information system or to paper, to the source of the image, either near the workstation or at some remote location, or anywhere else the radiologist desires. E-mail over the internet, including copies of images seen, may be sent automatically from the workstation. In the event that dictation of the diagnosis is necessary, the workstation can still capture information concerning which specific images have been read (as well as the rest of the standard information required) and make a notation to see the dictated report. As with the normal diagnosis, this information can be quickly routed to the appropriate destination. In addition, typed comments can be entered and routed to the appropriate destination. If a dictation has been made, an audio dictation file can be stored and routed. If required, the workstation can direct specific images to be printed, along with notes that the reading physician may wish to make. Like the other items of the diagnosis, this can be routed and/or e-mailed as desired.

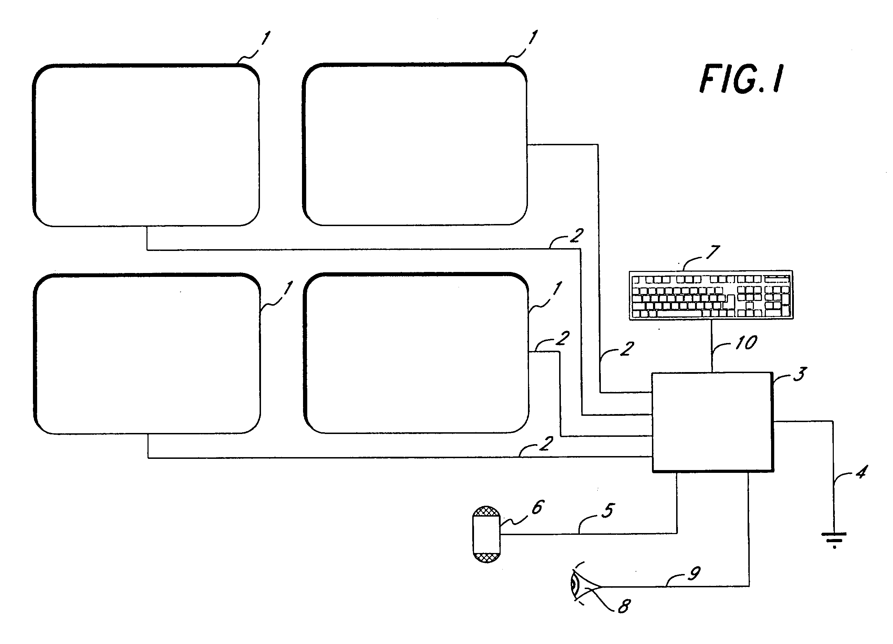

Referring first to Fig. 1, a schematic of a viewing station according to an embodiment of the present invention is provided. In the preferred embodiment, there is a viewing station comprising one or more video monitors (or other display means such as LCD, plasma panels, or projection systems, etc.) **1** coupled via cables **2** to a computer system **3**. The video monitors **1** and the computer system **3** may alternatively be connected via any type of data communication pathway such as wires, microwave, light wave, sound wave or radio wave. The image(s) to be viewed arrive at the computer controlling the viewing station either over a communication line **4** or on removable media such as a diskette or a removable hard drive. Once the viewing physician logs onto the viewing station (by entering a password or through another identification method such as a voice print, retina pattern or finger print), the viewing station presents the first patient's studies. Other manual input devices such as keyboards, foot switches, and pointing devices, *e.g*., trackballs, trackpads, computer mice, drawing pads, touch screens, light pens and the like, are used for changing the system or user profiles, or system command and control protocols (software) and are represented by item **7**, which represents all types of possible manual inputs, on FIG. 1. These devices as represented by item **7**, are connected via a cable **10** to the computer system **3** which controls the viewing station. Alternatively, these devices **7** may be connected to the computer system **3** via any type of data communication pathway such as microwave, sound wave (ultra sound), light wave or radio wave.

The following additional devices may also be connected to the computer **3**, depending upon the configuration desired by the client: (a) phone line (for internet access or remote diagnostics and/or control), (b) a network connection that may be different from **4**, (c) speakers to provide audio instructions, (d) a video monitor calibration tool, (e) archival storage, (f) backup devices, (g) printers, etc.

Although it is not always stated explicitly, the manual input to a modern graphical user's interface of the type described below can be roughly divided into "data input" versus "pointing". Generally, data input uses a keyboard or similar device to actually input "random" information, both software commands as well as actual data upon which the software operates. However, much of the data input function has been simplified through the use of menus which list commands or even preselected data for automatic input. Generally, a pointing device such as a mouse or trackball is used to select the desired menu item by "pointing" to it. Often the control functions are redundant so that they can be entered in a number of alternate and convenient ways. For example, while actual data is typed in or selected from a menu (where the data choices are constrained) and control functions are selected from a menu by pointing, control functions can usually also be selected by a "keyboard shortcut" (a special sequence of key strokes) and, in some systems, the entire command can be typed in. In the case of image manipulation, such as with the workstation of the present invention, it is usual to apply software "tools" to all or portions of an image (for example to alter contrast or rotate the image, etc.). The "tools" can be considered commands and are selected by any of the usual methods to issue commands. The pointing device is then used to select an area of interest in the image on which area of interest the selected tool will operate.

The following commands are examples from a working software system described in copending application 09/339,427:

| **Command** | **Action** |
|---|---|
| Rotate | Rotates the image on the monitor 90 degrees clockwise. If additional rotations are required, this command may be invoked successive times. |
| Invert | Reverses light and dark on the image. All shades of gray are similarly inverted (dark gray becomes light gray). |
| Flip Image | Flips the image from left to right on the monitor. This is useful if an image is scanned backwards (film emulsion facing the wrong way). |
| Switch with <number> | Move the image from one monitor to another and the image from the other monitor to the first monitor. |
| Zoom | Increase or decrease the image size. " Zoom In" increases the image size, while "Zoom Out" decreases the image size. (This item is not available for selection from the menu, but is a voice command.) |
| Zoom to 100% | Images with resolutions larger than the screen can display are originally reduced so that the whole image is displayed. This command Zooms in on the image so that one pixel of the original image is displayed on one pixel on the monitor. |
| Pan | Move the image around so that other parts may be displayed. This feature is only used after the image has been zoomed, since the whole image will automatically fit on the monitor before that. The image may be panned up, down, left or right. |
| Window/Level | Window defines the darkest and lightest gray that will be shown. A 16-bit image can have over 64,000 shades of gray. If a modality can only generate shades between 10,000 and 20,000, the window may optimally be set 10,000 (the difference between the maximum and minimum). Level defines the mid-point of the window. In the above example, the optimal level may be 15,000. Modalities may send optimal window/level values for images, which supercedes the prior settings. However, both window and level may be adjusted by the reading radiologist. By adjusting Window and Level, physicians can see details that may not be obvious with a static image. |
| Cine or Stack Animation | Where the images are taken sequentially in time, they may be displayed in an animation to resemble that time-sequence. This allows viewing of dynamic events such as angiograms and also allows for quick viewing of MRI slices. |
| Measure | Items on the image may be measured in one of three ways: |
| | Distance - If the scale has been provided to the system, the measurement will be displayed in centimeters (or millimeters, if appropriate). If there is no scale associated with the image, a known-length reference scale may be measured to establish a centimeter scale. |
| | Angle - The angle between two lines drawn on top of the image is displayed. |
| | Hounsfeld Units- A measure of density, relevant to CT images. |
| Display DICOM (or other) data elements | Shows additional data elements provided with the image. DICOM allows hundreds of different pieces of information to be passed with an image (ranging from patient name, modality taking image, resolution of image, optimal window/level settings, acquisition date and customized information). |
| Subtraction of two images | Where images are taken from the same position over time, it may be useful to subtract the common elements of each image to allow the physician to view the changes. |
| Go to next patient | There are several methods to advance to the next patient. Each method does something different to the current patient's study. |
| Skip Patient | Leaves current patient's study alone and simply advances. If the current patient's study was unread before this action, it will remain unread. |
| OK - Next | Marks current patient's study as read and notes that there are no acute abnormalities found. This information may be printed immediately, routed to a network for remote printing or reporting at a remote station, routed to a dictation pool, held until the reading session is over or handled in other appropriate methods. |
| Remarks | Marks current patient's study as read and notes that there is dictation or typed or dictated notes associated with this diagnosis. This information may be printed or played (if audio dictation is made through the system) immediately, routed to a network for remote printing or reporting at a remote station, routed to a dictation pool, held until the reading session is over or handled in other appropriate methods. |
| Previous | Re-displays previous images. |
| Worklist | Displays unread and read studies and allows non-sequential access to any patient study. |
| Print Images | Prints image to specified equipment. |
| Options | Allows access to user or institution specific options. Allows for entry of new physicians and customization of features and displays. |
| Freeze | Allows a single monitor to keep its image while Advance Images updates the rest of the monitors with other images. |
| Unfreeze | Remove a "Freeze" command from the active monitor. |
| Connection Reset | Resets the TCP/IP connection to the RIS or other database that holds patient demographics and previous reports. |
| Update Report | See if any new information has arrived on the or other RIS database, and if so, get and display it. |
| Email | Enables the sending of information, such as patient information, images, reports, voice dictation, via email. |
| Logout | Closes image review application. |
| Reset | Redisplays the image to the original data set |

Many of the commands can be accessed through menus. To avoid the physician having to move his/her eyes from the image being studied, the menu can be displayed at the cursor location as opposed to at the edge of the screen as in many graphical user interfaces. The menu can be displayed in a compressed format or an extended format, which contains more features. This mode of display can be preset as a physician preference. The expanded menu might consist of the following commands, any of which can be selected with the pointing device:

| | | | | |
|---|---|---|---|---|
| OK -- Next | Advance Images | Zoom to 100% | Pan | Window/Level |
| Remarks | Skip Patient | Rotate | Flip Image | Less |
| | | | | Features/Extra |
| | | | | Features |
| Previous | Work List | Measure | Subtract | Invert |
| Options | Print Image | Cine/Stack | Image Details | Reset |
| Logout | Email | | | Freeze/Unfreeze |

The workstation's controls have been designed to be simple to use and remember. Physicians are not required to learn complicated keystroke sequences to perform the functions they desire. Generally, redundant methods of manual input are supplied. For example, a trackball (or mouse or similar pointing device) can allow the physician to move the cursor to any location on any monitor. Alternatively, touch screens or light pens can be provided to permit manual cursor control. Gaze control based on the physician's head or eye position can replace more traditional pointing devices. Once the pointing device has set the cursor location, commands may be issued that will effect that location. Commands can then be issued using the pointing device and menus or by means of the keyboard or by means of voice control or any combination of these methods.

The workstation responds to spoken commands. A microphone is connected to the system which allows the voice command to be interpreted by a speech recognition software module and passed to the display and control modules as if invoked by the pointing device. To increase recognition accuracy, only appropriate commands are included in the dictionary of words to be recognized. In this way, the system "concentrates" on matching only a small subset of possible voice commands. For example, when the system is in the Cine mode, only commands related to Cine are active. The applicable voice commands and subcommands are:
- Select *<screen number>*
- Switch with *<screen number>*
- OK Next
- Remarks
   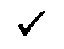 OK
   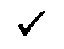 Cancel

   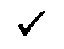 Playback
   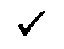 Stop
- Advance Images
- Skip Patient
- Previous
- Worklist
   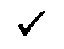 Cancel
   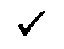 Show Unassigned
   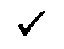 Delete Mode
   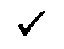 Select Mode
   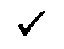 Query
      ◆ Name
      ◆ ID
      ◆ Query
      ◆ Get Selected
      ◆ Exit
      ◆ Up Query Level
      ◆ Clear
      ◆ <patient's name>
   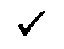 OK
   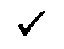 Delete
   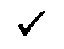 <patient's name>
- Turn Voice Off
- Turn Voice On
- Reset
- Window Up
   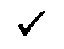 Stop
- Window Down
   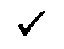 Stop
- Level Up
   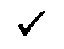 Stop
- Level Down
   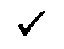 Stop
- Window/Level
   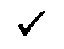 Window Up
      Stop
   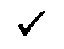 Window Down
      Stop
   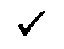 Level Up
      Stop
   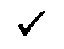 Level Down
      Stop
   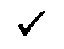 Optimize
   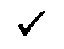 Contrast Equalize
   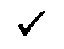 Preference <preference number>
   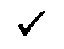 Reset
   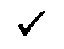 OK
- Flip Image
- Rotate
- Invert
- Zoom to 100%
- Zoom In
   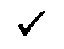 Stop
- Zoom Out
   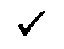 Stop
- Pan Up
   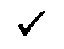 Stop
- Pan Down
   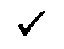 Stop
- Pan Left
   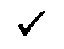 Stop
- Pan Right
   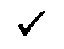 Stop
- Subtract
   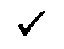 Original Frame
   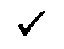 Frame to Subtract
   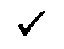 *<numbers - spoken using only 0-9, so thirty five is three five>*
   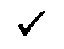 Optimize Image *(toggles on*/*off*}
   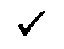 Cancel
   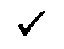 Show
- Measure
   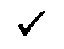 Distance
   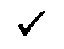 Angle
   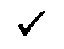 Hounsfeld Units
- Cine (or Stack, depending on the underlying modality)
   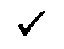 Start
   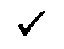 Stop
   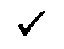 Continue
   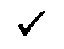 Zoom
   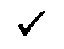 OK
   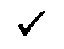 Adjusted
   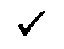 Modality
   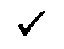 Preference <preference number>
   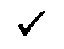 Backward
   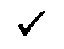 Forward
- Image Details
   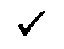 OK
   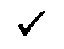 Page Up
   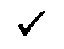 Page Down
- Options (all actions under Options currently require pointing device input)
- Print Image
- Extra Features
- Less Features
- Freeze
- Unfreeze
- Connection Reset
- Update Report
- Page Up
- Page Down
- Email
- Logout

To keep the reading physician's attention focused on the images, when possible, the system will respond with audio feedback indicating the status of the system. This audio feedback may be presented through speakers (with adjustable volume control) or through an earphone or headset and is available even when the physician does not use Voice Commands. If the audio feedback is presented through a headset, the same headset can conveniently be part of the gaze control system. The audio feedback may indicate that an action has been taken ("patient studies read - no dictation", "skip studies"...) or why an action can't be taken ("more images to read", "can't pan up", "no cine available"...). Any type of alerts can conveniently be presented through the audio feedback system including information on the arrival of emergency, or "stat", cases that require immediate information. Again, the goal is to present vital information without forcing the physician to divert his or her eyes from the image being studied.

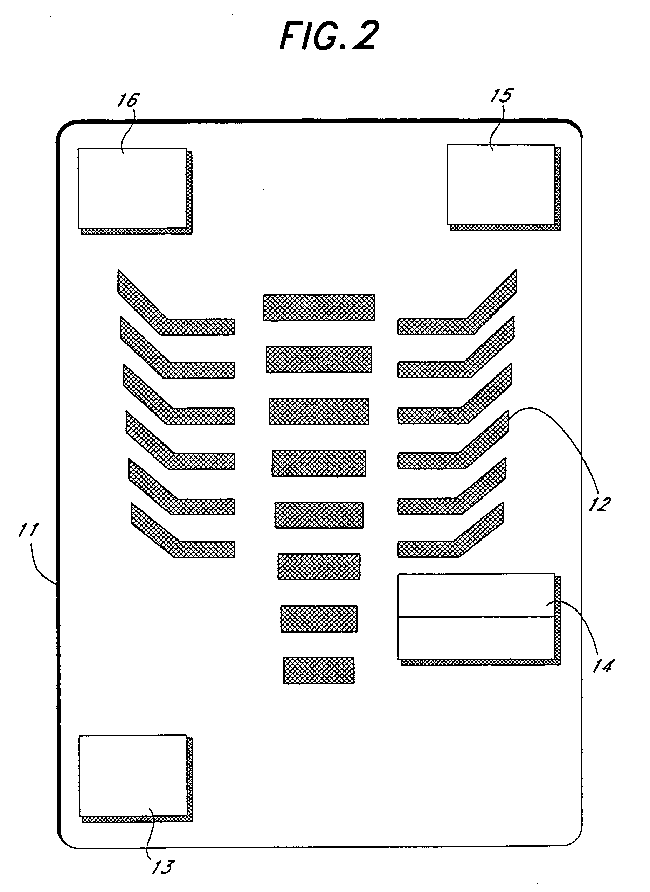

These features are illustrated on the screen interface of FIG. 2 as will be described below. Although these commands can be issued using manual techniques, it is desirable to limit distraction of the reviewing physician. Therefore, the system is configured to accept input of these, and other commands by voice command. To achieve this voice input, as shown in FIG. 1, a microphone **6** is connected via a cable **5** to the computer system **3** which controls the viewing station. Alternatively, the microphone **6** may be connected to the computer system **3** via any type of data communication pathway such as microwave, sound wave (ultra sound), light wave or radio wave. The physician issues a verbal command into the microphone **6**, which transmits it to the computer controlling the viewing station for interpretation and action. The computer employs voice recognition software to interpret a restricted list of words as commands. By limiting the number of words allowable as commands it is possible for the recognition system to recognize a wide ranges of voices and speech styles without requiring computer training for each user. After the voice input is translated to commands, and the commands execute, the resulting image(s) are then transmitted from the computer system **3** to the video monitor(s) **1**.

When a physician studies actual hard copy films, it is a simple task to actually manually grab the film and look in detail at a portion of the image-for example with a magnifying aid. It is also possible to mark a portion of a film for future study or by inspection by other experts. This can be accomplished either by making a sketch of the film or by actually applying a marker to the film surface. These simple tasks become much more complex with the advent of a digital image viewing system. To identify a portion of an image it is generally necessary to use a mouse or similar pointing device to outline or otherwise identify the desired image portion. As pointed out above, many physicians are not familiar with such pointing devices. Even when the physicians are used to pointing devices, use of such devices often slows the workflow unacceptably. It will also be apparent that identifying an image portion does not lend itself well to voice control. Unless quadrant numbers or some other identifiers are presented on the image, voice control of image portions is virtually impossible. Even with such identifiers the process is cumbersome.

A more natural way of handling the problem of image portion selection is to allow the physician's gaze to do the work. That is to say, a person will naturally gaze or stare at the important portion of an image. If the system is able to determine at which region the physician is gazing, that region can be automatically selected. At least two technologies are known for allowing the computer system to determine where the physician is gazing. The more complex method relies on actually determining the focal point of the physician's gaze. This is achieved through an image analysis system (e.g., a small video camera located with the video monitors to capture the physician's image which is sent to the computer system for analysis) which constantly analyzes the physician's head position and the position of the pupils of the physician's eyes. From these computations the system is able to decide which portion of the image is in the center of the physician's gaze. A simpler approach that yields the same information is to have the physician wear a headset or similar device that projects a beam of light (preferably invisible infrared light) towards the screen where one or more light sensors or camera devices detect the striking point of the beam. Other types of "gaze" determining devices are known in the art. The system automatically selects an image region surrounding the center of the physician's gaze. Alternatively, the deduced gaze center can be used to "steer" a cursor in a manner analogous to the use of a computer mouse or similar pointing device but without any use of the hands.

The present invention is directed towards non-manual control of an image viewing station. Voice command is a principal non-manual control method and is somewhat analogous to command and control by means of menus. However, voice control is not well adapted to "pointing" and as explained above, image manipulation benefits from the use of a pointing device. Therefore, it is advantageous to allow the physician's head or eye movement to control a non-manual pointing device to direct the location of a cursor or otherwise delimit a region of interest on the screen of the viewing station. This type of non-manual input is sometimes called " gaze control." Pointing by means of gaze control allows the reading physician to merely look at the portion of the image of interest and then issue the appropriate voice or other command to the viewing station which responds by enlarging the pointed to portion, or centering the pointed to portion, etc. This avoids the tedious manual or voice task of identifying a particular image portion to manipulate.

A gaze control system **8** is connected to the computer **3** through a data communication pathway **9**. The pathway **9** may be a simple cable but any other type of data communication pathway, as mentioned above in conjunction with the cables **2** and **5** can also be used. The primary function of the gaze control pointing device is to select images or image regions for the commands (voice or menu) to operate upon. However, it will be apparent that the gaze control pointing device can perform any of the traditional functions of a pointing device, *e.g*., selecting a command from a menu or selecting a button or switch in a dialog box, etc. Again the system is extremely flexible because of the nature of the non-manual command inputs provided. The voice control primarily issues commands-rather like a combination or a pointing device and a menu or typing in a command. However, the voice control can also invoke a menu or select a button-rather like a pointing device alone. Gaze control can select an image or image region like a pointing device and can issue commands by selecting them from an item. The non-manual functions are complementary and somewhat interchangeable. The physician is able to mix or match as he or she finds most convenient. Further, traditional manual input (keyboard and pointing devices) remain fully functional and can also be intermingled with the non-manual controls. Not only does this provide the utmost flexibility for the physician, it also provides great convenience for a disabled or handicapped physician. For example, a physician with limited hand or arm mobility can readily use voice and gaze control to rapidly process a large number or images.

The viewing station can display not only images, but also various reports on patient data. Such reports may come from a Hospital Information System or a Radiology Information System or other storage database as well as from the referring physician. By being able to cross check test and other data on the patient the reviewing physician can often confirm or extend his or her diagnosis. With less versatile systems the physician would have to phone or otherwise contact a laboratory or Hospital Information System to confirm a possible diagnosis. This interrupts the work flow and considerably slows the whole process.

Referring now to Fig. 2, a typical screen interface according to an embodiment of the present invention is provided. The figure shows one example of typical user interface, but is not intended to limit the invention. The viewing area as represented by a monitor **11** is used to display an image **12** and basic information **13** about the patient including the patient's case or institution identifier number and information on the most recent command that has been issued by the physician to the system as well as the status of voice and gaze control and other available system control inputs. The system is designed to train the user as he or she operates the system. The viewing station notifies the user when an improper or illogical command has been detected and/or instructs the user on the proper use of a command. Warning can be by visual and/or audio means, such as feedback through a headset or speaker system, or a warning message or icon (flashing) on the screen. As part of the automatic education function a detailed command menu **14** that lists other available commands can be made to appear (by voice or manual means) anywhere on the screen. Generally, the overall command structure, either through voice or menu, is contextual. That is, the commands available at a particular instant are related to the present status of the image and system. For example, when the Window/Level command has been given, only commands related to the Window/Level function are available. That means that Zoom, Rotate and other commands will not be active. Other icons or word descriptions **15, 16** can be used to remind the user of the status of the overriding command structure being used (voice, manual control, gaze control or a combination of these), whether additional images are available or a variety of other status issues. In addition, other items can appear on the monitor that relate to the images. This could include, among other items, clinical reports that describe the reason for the study, reports from prior studies, and unverified reports that require verification.

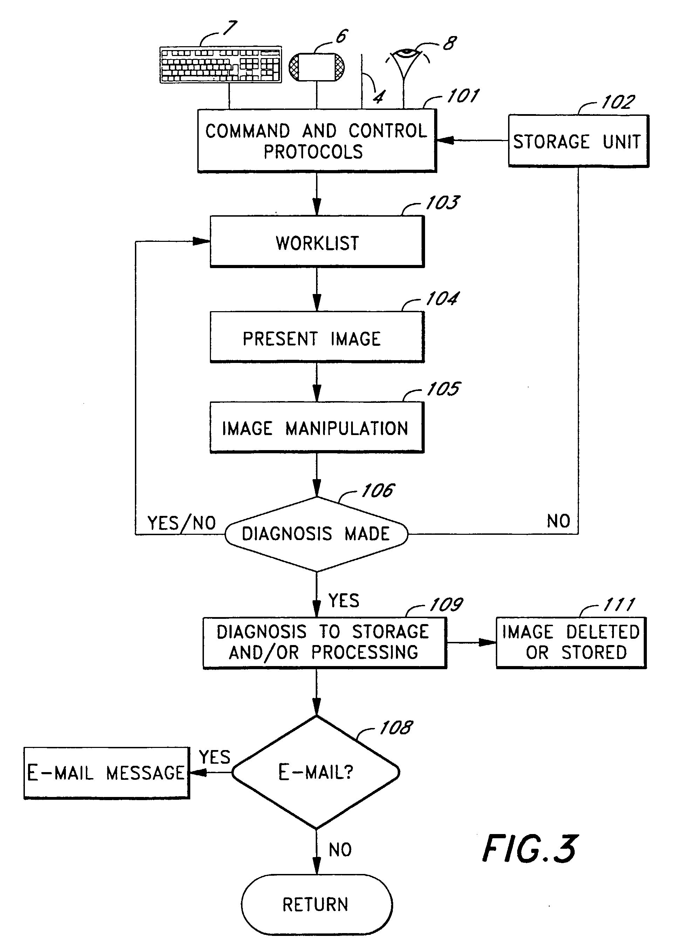

Referring now to FIG. 3, a typical flow diagram of the system according to an embodiment of the present invention is provided. Images are received by means as described in FIG. 1 and reproduced in FIG. 3 as item **4**. In step **101**, these images are processed through the command and control protocols and in step **102,** the images are temporarily stored in the storage unit. In step **103**, information about the image is sent to the worklist and included on this list based on either information sent with the image and/or by protocols stored in the command and control protocols. The system is connected via a data feed (FIG. 3, item **4**) and the image arrives by a means described in relation to FIG.1 and reproduced in FIG. 3, item **4**. The user controls the system via an input device (FIG. 3, items **6, 7**, and/or **8**). In its more complex application, the user log-in command is matched to that user's profile and to the appropriate worklist. In step **104**, the first set of images on the worklist is presented. In step **105**, using the image manipulation tools stored in the command and control protocols the images are reviewed by commands issued by the user. In step **106**, the user, at some point, may elect to make or not to make a diagnosis. This decision either sends the image being reviewed back to the storage unit and sends the work item either to the unread portion of the worklist (if no diagnosis is made) or to the finished work portion of the worklist (if a diagnosis is made). In step **108**, the user may elect to have the system send the diagnosis and/or the analyzed image(s) to a designated recipient by e-mail. Generally, the images are compressed to speed transmission. The recipient may be the requesting physician, a colleague who is aiding with diagnosis or providing a second opinion, a portion of the Hospital Information System (HIS), or an insurance company or some other entity that requires direct information regarding the diagnosis and its basis.

In step **109**, if a diagnosis is made, then information regarding the type of diagnosis and, in a preferred embodiment of the current application, the diagnosis itself in either audio or digital form is recorded for later processing. This processing could either be done within the system or sent to another system (*e.g*., part of the HIS). The information or the processed reports can be automatically forwarded by e-mail as mentioned above. Thereafter, in step **111**, the image will either be deleted from the system (typically to be long-term archived outside the viewing station) or stored for a short period of time on the system. These choices are determined by the amount of storage available within the system and the work flow of the physicians using the system. For example, if the using physician or another physician is expected to reassess the image in the near future, it is preferable to keep the image in local storage (on the workstation). Of course, it is possible to add a long-term archiving function to the present invention. This is especially convenient if the physicians habitually refer to old images in making their diagnoses or may serve as the system-wide archive for old digital images.

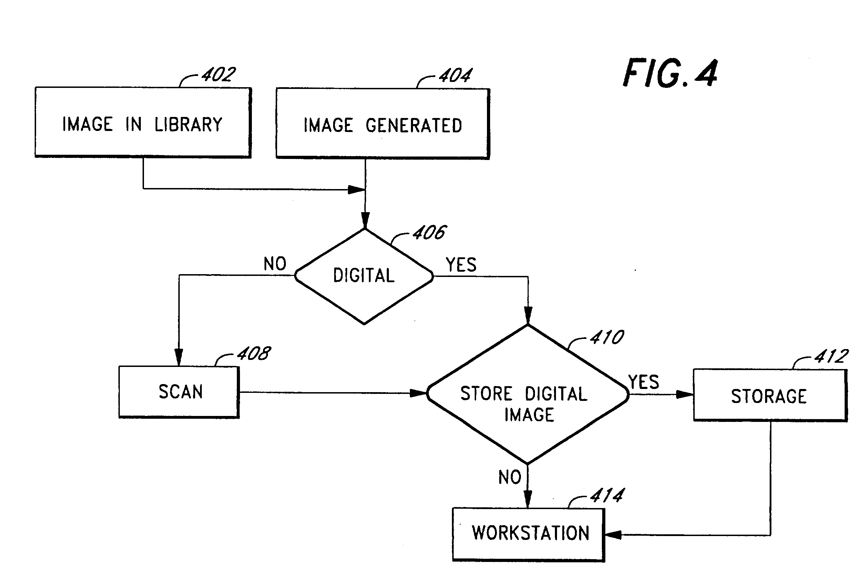

Referring now to FIG. 4, a drawing illustrating a workflow on a preferred system according to an embodiment of the present invention is provided. A further detailed description of the system of the inventive workstation is provided. This workstation is derived from a commercial product (CATELLA, trademark of AMD Industries, LLC). Images may be provided from a library as in step **402**, or the images may be generated, as in step **404**. In step **406**, it is determined if the images provided are digital. If the images are not digital, the system goes to step **408** in which the images are scanned to generate a digital image. In step **410**, the system determines whether to store the digital images. If the digital images are to be stored, the system goes to step **412** and the images are stored until they are read. The images may be sent to an archiving system or a film library for storage. Whether the digital images are to be stored or not, the images go to step **414**. These steps will be described in more detail below.

The inventive workstation stores the images until they are viewed by the physician. However, permanent storage (up to seven years and in some cases for the life of the patient) is usually required for medical images. While the workstation can store images for a short period of time after reading, the primary long-term storage of the images generally occurs elsewhere. The workstation can be connected to an archiving system or to a film library for such storage. The capacity of the commercial version workstation in its standard form (the standard system uses hard disk storage to keep retrieval speeds fast) is 200 2k x 2k images (typical chest X-ray) or 8,000 512 x 512 images (typical MRI). The system may be expanded to hold over 10,000 2k x 2k images or over 400,000 512 x 512 images. To prevent exceeding its storage capacity, the system can be configured to automatically delete images based upon various criteria including date of last access. Thus, the last used images are the first to be deleted when the system is starting to fill up. Other automatic deletion criteria can be set, including source of the image and confirmation that the image has been properly stored in a permanent storage location prior to deletion.

Having thus described preferred embodiments of a non-manual control of a medical image display station, it should be apparent to those skilled in the art that certain advantages of the within system have been achieved. It should also be appreciated that various modifications, adaptations, and alternative embodiments thereof may be made within the scope and spirit of the present invention. The invention is further defined by the following claims.

## Claims

1. A method for controlling a medical image digital display system using non-manual commands, comprising the steps of:
providing a medical image digital display system having a plurality of digital image displays (1);
providing means (3) for detecting and decoding non-manual commands;
deriving a control signal from said non-manual commands; and
applying said signal to the medical image digital display system to control the system in accord with the non-manual commands,
**characterised in that** control of the system with the non-manual commands includes selection of one of the plurality of image displays (1) and includes control of an integral dictation device.

2. The method of Claim 1, wherein said non-manual commands comprise voice (6) and/or gaze control (8).

3. The method of Claim 1, wherein head and/or eye motion sensing hardware (8) and/or software is incorporated into the medical image digital display system to allow head or eye movements to control the medical image digital display system.

4. The method of Claim 1, further comprising displaying non-image information (13) including at least one of patient data, history, test data and information.

5. The method of Claim 1, wherein said deriving step further comprises recognizing a spoken voice command and translating said spoken voice command into said signal.

6. The method of Claim 1, further comprising using at least one of a keyboard (7), pointing device and a footswitch to control the medical image digital display system.

7. The method of Claim 6, wherein the pointing device is selected from the group consisting of a trackball, a trackpad, a computer mouse, a touch screen, a light pen, drawing pad and a gaze detection system (8).

8. The method of Claim 1 further comprising the step of allowing the user to decide whether to make a diagnosis based on an image displayed by said display system; and if a diagnosis is made, storing information regarding the diagnosis, wherein the information regarding is processed by the dictation system under control of said signal.

9. The method of Claim 8 further comprising a step of transmitting information about the image displayed and/or the diagnosis.

10. The method of Claim 9, wherein the step of transmitting utilizes the internet.

11. An apparatus for controlling a medical image digital display station, wherein said digital display system includes means for receiving images, means for displaying received images (1) and means for controlling (3) said displaying of said received images in response to non-manual inputs from a user, **characterized by** having means for controlling which responds to voice commands and further controls a dictation system for taking dictation.

12. The apparatus of Claim 11, wherein said means for controlling further comprises means (8) for controlling said displaying in response to a gaze of the user.

13. The apparatus of Claim 11, wherein the means for receiving images further comprises a transmission media that includes at least one of a wire, cable, microwave, radio wave, light wave, sound wave, and a physical medium.

14. The apparatus of Claim 13, wherein said physical media further comprises at least one of a floppy disk, a removable hard disk, a CD ROM, and a tape.

15. The apparatus of Claim 11, wherein said displaying means further comprises at least one of display monitors, projection systems, and printers connected to a computer system.

16. The apparatus of Claim 11, wherein said means for controlling said displaying of said received images further comprises a microphone (6) connected to a computer system capable of recognizing voice commands.

17. The apparatus of Claim 11, wherein the dictation system comprises a computer system capable of recording a user's voice and converting said recorded voice to text.

18. The apparatus of Claim 11, wherein said means for controlling said displaying of said received images further comprises manual means including at least one of a keyboard (7), a trackball, a mouse, a foot pedal, a drawing pad and other manual input means.

19. The apparatus of Claim 11, further comprising means for displaying non-image information (13).

20. The apparatus of Claim 11, further comprising means for receiving head or eye control commands to point to a location on a display means for indicating a current location of interest and means for controlling a movement of the current location of interest with head or eye changes.

21. The apparatus of Claim 11, further comprising visual means for notifying and instructing a user.

22. The apparatus of Claim 11, further comprising audio means for notifying and instructing a user.

23. The apparatus of Claim 11, wherein the means for displaying comprises a plurality of displays (1) for visually displaying images and the means for controlling allows selection of one or more of said displays.

24. The method of Claim 11 further comprising the step of providing a long term archiving function for storing images.

25. A machine-readable recording medium for use in a medical image digital display system having a central processing unit for controlling the medical image digital display system by using non-manual commands, the medium including instructions executable by the central processing unit for causing the medical image digital display system to perform a method comprising the steps of receiving images in an electronic form, receiving non-manual commands from a user, processing said images through command and control protocols according to the non-manual commands, displaying said processed images and allowing the user to decide whether to make a diagnosis based on said processed images **characterized by** including using the non-manual commands to control a dictation system for processing the diagnosis.

26. The medium of Claim 25 further comprising instructions for transmitting information about said processed images and/or said diagnosis.

27. The medium of Claim 26, wherein the step of transmitting utilizes the internet.
